Europäisches Patentamt

European Patent Office

Office européen des brevets

(19)

(11) Publication number: **0 543 765 A1**

## (12) EUROPEAN PATENT APPLICATION

(21) Application number: **92500139.8**

(22) Date of filing: **30.10.92**

(51) Int. Cl.⁵: **A61L 25/00, A61K 6/033**

(30) Priority: **22.11.91 ES 9102606**

(43) Date of publication of application:
**26.05.93 Bulletin 93/21**

(84) Designated Contracting States:
**BE CH DE FR GB IT LI NL**

(71) Applicant: **Boltong, Maria Gertruda**
**Mezenlaan 12**
**NL-6581 CM Malden (NL)**

(72) Inventor: **Boltong, Maria Gertruda**
**Mezenlaan 12**
**NL-6581 CM Malden (NL)**

(74) Representative: **Aguilar Forment, Domènec**
**c/o Aguilar & Revenga C. Consell de Cent 415,**
**5.o-1.a**
**E-08009 Barcelona (ES)**

(54) **Process for the preparation of calcium phosphate cements and their application as bio-materials.**

(57)    The invention refers to the use of a series of calcium ortophosphate cements as biomaterials, starting from a number of components by grinding and sieving to obtain the adequate grain size, the needed quantity being weighed and the blend of powder products being put into recipients to be shaken up for its homogeneization, the mix being, placed afterwards, in the desired portions, into a container to be supplied to the specialists, together with the capsules containing water or a water solution.

EP 0 543 765 A1

Jouve, 18, rue Saint-Denis, 75001 PARIS

## SUBJECT OF THE INVENTION

The present invention refers to a process for the preparation of calcium phosphate cements and its application as bio-materials, which obvious objective is to allow its use in bone surgery and odontology.

## FIELD OF THE INVENTION

This invention corresponds to the field of the industrial manufacture of bio-materials for bone surgery and odontology.

## DESCRIPTION OF PRIOR ART

Calcium and ortophosphate ions are regulary contained in body fluids and in the mineral substance forming part of bones, dentine and dental enamel.

These are calcium ortophosphates, which additionally contain sodium, magnesium and carbonates.

Sintered hydroxyapatite and calcium tri-phosphate, as well as other synthetic calcium ortophosphates, have been implanted to a number of test animals, as well as to human beings, having evidenced to be bio-compatible.

It has been observed from the use of this kind of materials that they are bone growth promoters, that is, after its implantation in bone tissue they promote their own growth.

A negative aspect of calcium sintered ortophosphates for its use in surgery is that they have to be moulded previously to the intervention, while ortophosphate cements do not show this disadvantage, because of its cement nature which allows them to be moulded by the surgeon during the intervention.

At present the existence of solids corresponding to the system $Ca(OH)_2$-$H_3PO_4$-$H_2O$ at room or physiologic temperature is already known.

Cement formulations are also known for the calcium ortophosphate system.

Finally, other formulations are known from other inventions, for which reason they are excluded from the present disclosure.

Concerning the solids belonging to the $Ca(OH)_2$ -$H_3PO_4$-$H_2O$ system at ambient temperature or physiological temperature, a table is included in the following in which the solids which may be formed in this system at ambient or body temperature are described.

Given the fact that pH in bone in the vecinity of osteoclasts, osteoblasts and other osteocites may vary in a range approximately comprised betwen 5 and 8, it has been observed that the final result of the solidification or setting reaction in a calcium ortophosphate cement will generally adopt the CDA form, which in physiological conditions will incorporate sodium and carbonate ions.

In bloodstream the apatites $Ca_{8,5}Na_{1,5}(PO_4)(CO_3)_{2,5}$ or NCCA and $Ca_9(PO_4)_{4,5}(CO_3)_{1,5}(OH)_{1,5}$ or HCDHA may be formed by precipitation.

Given the fact that magnesium ions may be present, the final result may consist in magnesium whitlockite or MWH.

PHA could also result by the action of some fluorides.

In each of the above cases, the intermediate formation of DCPD or OCP is also possible.

In case that in a formulation of calcium ortophosphate some solidification reaction appears, it will be due to the framework constituted with the recently formed crystals.

As consequence, the best results which can be expected from a calcium ortophosphate cement as concerns mechanical properties consist in that this cement may come close to the mechanical characteristics of hard dental gypsum or other hard gypsum formations in which the framework of similar crystals (dicalcium sulphate dihydrate) is the mechanism for the solidification or setting.

The solids appearing in the system $Ca(OH)_2$-$H_3PO_4$-$H_2O$ at ambient temperature or physiological temperature (1) are those shown in following Table 1:

## Table 1

| Abbreviation | Ca/P | Formula | Formation conditions and relative stability |
|---|---|---|---|
| MCPM | 0,5 | $Ca(H_2PO_4)_2H_2O$ | Established below pH 2 approximately. |
| Brushite or DCPD | 1,0 | $CaHPO_4x2H_2O$ | Established at pH comprised between 2 and 4, rapid nucleation and growth up to pH > 6,5. |
| OCP | 1,33 | $Ca_8(HPO_4)_2(PO_4)_4x5H_2O$ | Rapid nucleation and growth in pH 6,5 and 8, more stable than DCPD or ACP in the same range. |
| ACP | 1,5 | $Ca_3(HPO_4)_2xH_2O$ | This substance appears as a first stage when the precipitation takes place at high concentrations with pH comprised between 4 and 8, however it spontaneously transforms into DCPD, OCP or CDA. By any means, by the incorporation of magnesium ions it will transform into magnesium whitlockite NWH which is as stable as CDA. |
| CDA | 1,5 | $Ca(HPO_4)(PO_4)_5OH$ | This calcium poor apatite does not spontaneously precipitate, however it has DCPD or OCP as precursors. By any means, it may rest indefinately in metastable balance with aqueous solution. |
| PHA | 1,67 | $Ca_{10}(PO_4)_6(OH)_2$ | The precipitated hydroxyapatite is the most stable calcium ortophosphate. It only precipitates above pH 8. By any means, its nucleation with a low pH maybe started by fluoride ions. |
| ---- | ∞ | $Ca(OH)_2$ | This solid is stable only with a high pH in absence of ortophosphates. |

Concerning cement formulations for the calcium ortophosphate system, any water based or water solution based cement as intermediate fluid medium, contains the following components:

a).- An acidic component which may emit H$^+$ ions which may be converted into a solid salt by reaction with disolved ions.

b).- A basic component which by reaction with the emitted $H^+$ ions may be able to emit the dissolved cations and that by this reaction will become a more or less stable and neutral gel or solid acid. It is also possible that the acidic component and the basic component react together giving ground to only one solid component.

c). - Water or a water solution in which both the acidic aid the basic components may dissolve working as a reaction medium.

d).- Eventually, accelerators, inhibitors or modifiers.

For the formulation of calcium ortophosphate cements the choice is not limited to the solids appearing in the aforegoing table.

Other solids may be incorporated into similar calcium containing components, specially those formed at high temperatures.

In the following Table 2 some relevant solids which may be prepared at a high temperature have been shown.

Table 2

| Abbreviation | Ca/P | Formula | Formation conditions and relative stability in water and ambient or physiologycal temperature |
|---|---|---|---|
| DCP or monetite | 1,0 | $CaHPO_4$ | Formed by precipitation at a high temperature, it is somewhat more stable than DCPD. |
| β-TCP | 1,5 | $Ca_3(PO_4)_2$ | Formed by heating to temperature up to 1.189 °C. More stable than DCPD and OCP but less stable than CDA in the 6 to 8 pH range. |
| α-TCP | 1,5 | $Ca_3(PO_4)_2$ | Formed by heating to over 1.180 °C and rapid cooling. It is less stable than DCPD and that OCP. |
| SHA | 1,67 | $Ca_{10}(PO_4)_6(OH)_{2-2x}O_x$ | Sintered hydroxyapatite. It is formed by heating in the 700 to 1.440 °C range. It is so stable as CDA. |
| TTCP | 2,0 | $Ca_4(PO_4)_2O$ | Formed by heating starting from 1.500 °C. It is less stable than SHA, CDA, DCPD and OCP in slightly acidic medium. After 450 °C it is less stable than $Ca(OH)_2$ and therefore it is to be expected that it will be more reactive. |
| ---- | ∞ | CaO | Formed by heating CaCO3 beginning at 450 °C. |

Additionally, to accelerate or to control the time necessary for setting or to improve the mechanical properties of calcium ortophosphate cements, it will be possible to use water solutions containing phosphoric, malonic, lactic, citric or other organic acids which are present in body fluids.

Given the fact that both the body fluids and the minerals contained in bones comprise also sodium carbonates, nagnesium, sulphates, hydrocloric and fluorhidric acid, other components and specially those mentioned in the following Table 3 may be also considered to fall within the calcium ortophosphate cement formulations.

In next Table 3 a relation of solids will be shown which are eventually relevant for the formulation of calcium ortophosphate cements, possibly appropiate as accelerators, inhibitors or even as substitute reagents for the acidic or basic components or as reaction products for the reagents which constitute a cement.

4

### Table 3

| Compounds which contain | Formulae |
|---|---|
| Sodium | * $CaNaPO_4$ (α or ß form), $Ca_{10}Na(PO_4)_7$ (α or ß form) $Ca_{8,5}Na_{1,5}(PO_4)_{4,5}(CO_3)_{2,5}$<br>* $NaF$<br>* $Na_2CO_3$, $Na_2SO_4$, $NaCl$, Na ortophosphates |
| Potassium | * $CaKPO_4$, $Ca_9K(PO_4)_5(CO_3)_2$<br>* $KF$<br>* $K_2CO_3$, $K_2SO_4$, $KCl$, K ortophosphates |
| Magnesium | * $Ca_4Mg_5(PO_4)_3$, $MgHPO_4$, $Mg_3(PO_4)_2 x 4H_2O$<br>* $MgF_2$<br>* $MgCO_3$, $CaMg(CO_3)_2$, $MgSO_4$, $MgCl_2$, $MgO$, $Mg(OH)_2$ |
| Zinc | * $CaZn_2(PO_4)_2$, $Zn_3(PO_4)_2 x 4H_2O$<br>* $ZnF_2$<br>* $ZnCO_3$, $ZnSO_4$, $ZnCl_2$, $ZnO$, $Zn(OH)_2$ |
| Calcium | * $CaSO_4$, $CaSO_4 x 2H_2O$, $CaSO_4 x 1/2 H_2O$<br>* $CaF_2$, $Ca_{10}(PO_4)_6F_2$<br>* $CaCo_3$, $CaCl_2$, $Ca_2PO_4Cl$, $Ca_{10}(PO_4)_6Cl_2$ |
| Biopolymers | Proteins, peptides, proteoglicanes, glycose-aminoglicanes, carbohidrates, etc. |
| Organic acids | Citric acid, malonic acid, pirmic acid, tartaric acid, etc. |
| Inorganic acids | Phosphoric acid, etc. |
| Sintetic polymers | Polilactic acid, poligricolic acid, etc. |
| Growth factor | T.G.F.-ß, etc. |

Solid carbonates are less desirable because carbon dioxide which may form during the reaction may cause the structure to explode, that is, it may affect the structure.

The only advantage that the carbonate may bring about is that as a consequence, a solid structure may be formed which at the same time is porous, in which structure the bone may show some degree of growth.

Hydrates are less desirable as reactive components as during the setting reaction water may be formed, weakening the structure.

However, DFP or OCP as reaction products may be favourable because they bind with water so they may reduce the volume of the reaction products.

With the list of possibilities shown in tables 1 and 2, we arrive to the following components which are deemed to be appropriate, shown in decreasing acidity or basicity:

a).- Acidic components: MCPM > DCPD = DCP > OCP > ACP = $\beta$-TCP = $\alpha$-TCP = CDA.

b).- Basic components: CaO = Ca(OH)$_2$ > TTCP > PHA = SHA > $\alpha$-TCP = CDA = $\beta$-TCP = ACP.

The setting reaction may be accelerated and even induced by the addition of crystal seeds of CDPD, OCP, BTCP, CDA, PHA or SHA.

Table 3 contains as well a list of other possibilities which are physyologically acceptable to initiate, accelerate or to delay the setting reaction.

Peptids and proteins as well as proteoglycans may be used additionally as modifiers with the ain of obtaining a greater approximation to the composition of the bones.

Also composites with synthetic reabsorbable or non reabsorbable synthetic polymers may be formed, being of importance the fact that growth factors like TGF-B may be easily incorporated into these calcium phosphate based cements with the aim of stimulating the growth of the bone.

Among the formulations which have been disclosed until now and that obviously are excluded from this invention, formulations based on TTCP as basic component in combination with other calcium phosphates may be cited.

The inventors are Brown and Chow (2), being mentioned as acidic components DCDP, DCP, OCP, $\alpha$-TCP and $\beta$-TCP.

The reaction with DCPC which takes approximately one week, may be accelerated by the addition of HA, a 48% addition causing the setting time to diminish from 22 to 8 minutes.

The combination of TTCP with DCPP[3] was more successful.

By the addition of sone fluoride to the blend to prevent its nucleation it was observed that the reaction product consisted in PHA[3].

In this last combination the inventors found that the reaction was completed in 24 hours.

The inventors determined a compression resistence of 37 MPa[2,3] although other authors (4) indicate for the combination of TTCP with DCPD values comprised between 6 and 11 MPa. The porosity is of 50% (4).

Concerning the combination of MCPM with $\beta$-TCP the inventors are Mirtschi, Lemaitre and collaborators (5 and 6) observing for 2 minutes the setting in combinations with $\beta$-TCP.

The product of the reaction was TCPD and the tensile resistence varies between 0,2 and 1,1 MPa.

The tensile resistence was lowered by drying and ageing in water.

The addition of calcium pirophosphate, calcium sulphate, dihydrate or hemihidrate calcium sulphate increases the setting time up to approximately 10 minutes and the tensile resistence in dry state to about 3 MPa.

By any means, even in that case the resistence decreases with the ageing in a saliva solution (7).

The combination of $\alpha$-TCP with DCPD is also kuown having Monma and his collaborators (8) studied the hydratation of $\alpha$-tricalcic phosphate.

The velocity decreases with temperature and pH.

The final product was CDA and even at 60°C the setting time was of some hours and the porosity was of 60%. The compression resistence was of about 17 MPa and the tensile resistence reached 3 PMa. This combination is not adequate as a cement due to its long setting time.

Monma (9) studied also the hydratation and hardening of brushite and monetite.

The hardening was obtained by the addition of CaCO$_3$ and water.

The final products were OCP and carbonate containing apatite.

The porosity was of approximately 75% and the tensile resistence in dry state varied within the 0,1 to 1,5 MPa range.

Due to the fact that the setting time was of approximately 1 hour to a temperature comprised between 50 and 80°C, this combination was not adequate for clinical purposes.

Monma et al (4) are the inventors of a better performing formulation, by combining $\beta$-TCP with DCPC as reagents. They found the setting time to be comprised between 9 and 30 minutes.

The product resulting from the setting reaction was OCP which porosity was of approximately 50% and the compression resistence was comprised between 14 and 15 MPa.

Concerning the formulation based on HA, this powder product with the addition of between 2 and 4% of CaO and between 2 and 6% ZnO was mixed in a chitosan son that, as well known, is a peptide derived from chitine in a solution of malic acid.

The setting tine, measured by menas of an adherence test, varied from 2 1/2 to 20 minutes being the compression resistence in dry state of 2 MPa (10).

Concerning the formulations using collagen as modifier, $\alpha$-TCP or TTCP in powder form have been combined with an antigenic collagen solution to obtain hardened products, no information having been given on setting time.

The compression resistence in dry state was of approximately 15 MPa and it has been found that when the liquid medium contained citric or malonic acid the compression resistence in dry state increased up to approximately 110 MPa.

Given the fact that the products obtained in these formulations were not calcium phosphates, these materials must not be called calcium phosphate cements and no data have been given concerning its resistence on ageing in presence of humidity.

In an experiment carried out with test animals, a $\alpha$-TCP with citric acid was used (12).

A hystological investigation after three years demonstrated that Ca-citrate accumulated in the osteocites and that the $\alpha$-TCP was transformed into apatite.

Recently a new calcium phosphate bioactive cement has been developed by Nishimura et al. 1991 (18).

This powder compound is a $CaO-SiO_2-P_2O_3-CaF_2$ system glass.

The powder is blended with an amonic phosphate water solution for 1 minute, with pH 7,4, and a rate liquid/powder of 0,5.

The mix sets in 7 1/2 minutes at 20 °C and its compression resistence is of approximately 16 MPa. However, after its inplantation in the hindolimbar muscles of rats said resistence increased up to 70 MPa one week after its implantation.

## DISCLOSURE Of THE INVENTION

The process (or the preparation of calcium phosphate cements and its use as biomaterials according to the invention, provides by itself and obvious solution to the problems now existing in this field.

Particulary, to carry out this process for the preparation of calcium phosphate cements and its use as biomaterials, a number of pre-requisites have to be taken into account for the use of these calcium ortophosphate cements as biomaterials, which in the first place have to see with its field of application.

As a definition, we will only call calcium ortophosphate cements to the formulations in which the setting is provoked or accompanied by the formation of one or more calcium ortophosphate precipitates.

In the setting and surgical handling of this material, the use of calcium ortophosphate cements as biomaterials allows the moulding of these materials during the surgical intervention.

To prevent the formation of cleavages and fluxes this moulding operation has to be carried out while the cement mix shows visco-elastic properties.

The inventors have used Gilmore light water (13) to determine the end of the visco-elastic period as initial setting time I.

After setting time I there is a certain period in which the cement will not admit any filler, even in small quantities, to avoid the danger of originating cleavages. Even in applications of the type called load-less or devoid of load support, the surgical handling during the intervention will apply some load to the material.

The inventors have used Gilmore heavy water (13) to determine the time from the beginning of the blend (final setting time F) when the material is sufficiently strong to support the continuation of the intervention.

With the aim to maintain the intervention time within reasonable limits, the inventors have accepted F (or final setting time) = 60 minutes as maximum.

Concerning the setting process in relation with the contact with body fluids, in the process for the use of calcium ortophosphate cements as biomaterials, it has to be taken into account that these materials come into contact with the body fluids prior to reaching the initial setting time I.

Therefore, there is still another pre-requisite necessary for the application of calcium ortophosphate cements as biomaterials, consisting in that whenever a cement mix contacts a water solution or it is submitted to relative humidity of 100% before the initial setting time I, at a physiological temperature, these conditions being maintained for a certain period of time (for example, a minimum of 20 hours) it must harden as defined by the test carried out with Gilmore heavy water.

Certain minimum values for compression resistence and tensile resistence have been found as desirable even for load less applications. These values have been determined in the present state of the art for a maximum of approximately 36 MPa for compression resistence and approximately 6 MPa for diametral tensile resistence for calcium ortophosphate cements under ideal physical conditions.

However, for load supporting applications a compression resistence of at least 100 MPa would be necessary under real physiological conditions, according to experience in ortopedics, even after contacting body fluids or under 100% relative humidity.

Notwithstanding, the use of calcium ortophosphate cements as biomaterials must be limited to applications which donnot support loads until in future a higher resistence may be obtained.

However, it is thought that even for applications without load, a higher compression and tensile resistence will be, up to a certain point, desirable characteristics.

These characteristics may be used to rate the quality of calcium ortophosphate cements as biomaterials at least in the applications which donnot support loads.

Concerning the minimum resistence to breakage as a necessary pre-requisite for non supporting load applications for calcium ortophosphate cements as biomaterials, it has been observed from experience in ortopedics that a minimum breakage resistence is necessary for the use of biomaterials in applications which have to support loads.

For the time being, the inventor does not have any evidence that calcium ortophosphate cements be desirable for load supporting applications. However, when reaching a higher development, a minimum breakage resistence will have to be formulated.

Concerning the choice of stability "in vivo" or "reabsorption in vivo" as desirable characteristics for the use of calcium ortophosphate cements as biomaterials, the surgeons consider that for certain applications the calcium phosphate biomaterials should be more reabsorbible for applications "in vivo" and that for other applications they should be less reabsorbible.

According to recent investigations, the most reabsorbible calcium ortophosphates are those consisting mainly in β-TCP, considering that all other calcium phosphates, after a certain period of time, will be in a situation of low reabsorbility.

It is of vital importance for the surgeon to have the posibility of choosing between more or less reabsorbible calcium ortophosphate cements.

To implement this process, in the first place the components as they have been described, will have to be comminuted to obtain a grain size comprised approximately between 5 and 10 pM.

Afterwvards, the adequate quantities will be weighed, putting the blend of powder products into a bottle or recipient provided with ample mouthpiece, filling it up to a maximum of 25% of the total volume or capacity of the recipient.

Subsequently a shaking action will be carried out on the recipient by rotation of the same on a roller for and hour, eventually substituting this action for a vigorous manual shaking for 5 minutes.

Afterwards, the blend which has been obtained will be put in portions in bottles or recipients for its supply to surgeons or dentists together with capsules containing the desired quantity of water or water solution.

By last, the bottles and capsules with its contents will be sterilized.

For its use in hospitals or health centers the powder product and the liquid will be supplied together on glass plates or small tiles to obtain a paste after mixing for one minute.

In case of injectable cements, the paste will be put in the added injection and the surgeon or dentist will carry out the injection of the paste into the body.

In case of cements not to be injected, the paste will be applied to the body by means of a palette knife.

## BEST MODE TO CARRY OUT THE INVENTION

The process for the preparation of calcium phosphate cements and its use as biomaterials is implemented in a first embodiment with powder blends composed of up to four reagents choosen among the following:

-MCPM, DCP, DCPD, OCP, β-TCP, α-TCP, TTCP, CaO, Ca(OH)$_2$, CaCO$_3$, CaMgO$_2$, CaHPO$_4$, CaNaPO$_4$, Ca$_{10}$Na(PO$_4$)$_7$, Ca$_2$PO$_4$Cl and Ca$_{10}$(PO$_4$)$_6$Cl$_2$.

The composition of these powder blends was choosen in such form as to take after the composition of the products from the following reaction: DCPD, OCP, CDA, PHA, NCCA or HCDHA.

These powder blends contained crystal seeds of DCPD, SHA or PHA or alternatively, they did not contain any seeds.

This composition choice fulfills the above stated prerequisites concerning the field of aplication, that is, by definition, only will be called calcium ortophosphate cements those formulatious in which the setting is provoked or is accompanied by the formation of one or more precipitates of calcium ortophosphate.

Also, some quantities of these powder blends were mixed with mininum quantities of water giving a consistent paste as result.

Metal rings were filled with this paste and the initial setting time I and the final setting time F at 35 °C were measured with Gilmore water, with the objective of verifying whether the above stated requisite was fulfilled concerning the setting and surgical handling of the material.

One of these metal rings for this paste was stored in a relative humidity of 100% at 35 °C approximately and it was kept in this way for about 20 hours.

Subsequently is resistence was measured by means of Gilnore heavy water to verify that the requisite concerning setting and contact with body fluids which has been mentioned in the aforegoing, was duly fulfilled.

The blends of powder products appearing in the following table, both those which contained crystal seeds as those which did not contain then, fulfilled the requisites concerning the application field, the setting and surgical handling of the material and the setting and contact with body fluids.

In following Table 4 a list of examples will be given for calcium ortophosphate cements setting in 60 minutes or less, which maintain its hardness after being kept at least for 20 hours in 100% relative humidity. In the table:

Column 1 refers to the number of the compound.

Columns 2 to 5 refer to the reagents.

Column 6 refers to the Ca/P molar relation of the blend of reagents.

Column 7 refers to the type of crystal seeds.

Column 8 refers to the porcentage of weight of the seeds in the powder cement.

Column 9 refers to the initial setting time (I) approximately at 37 °C.

Column 10 refers to the final setting time (F) approximately at 37 °C.

Table 4

| 1 | 2 | 3 | 4 | 5 | 6 | 7 | 8 | 9 | 10 |
|---|---|---|---|---|---|---|---|---|---|
| 1 | OCP | Ca(OH)$_2$ | ---- | ---- | 1.50 | PHA | 40 | 25 | 55 |
| 2 | MCPM | CaO | ---- | ---- | 1.50 | PHA | 40 | 15 | 30 |
| 3 | MCPM | CaO | ---- | ---- | 1.67 | PHA | 40 | 8 | 20 |
| 4 | α-TCP | ---- | ---- | ---- | 1.50 | PHA | 40 | 20 | 60 |
| 5 | DCP | CaO | CaMgO$_2$ | ---- | 1.28 | ß-TCP | 40 | 5 | 18 |
| 6 | MCPM | TTCP | ---- | ---- | 1.50 | PHA | 40 | 20 | 60 |
| 7 | DCP | TTCP | ---- | ---- | 1.50 | PHA | 40 | 15 | 50 |
| 8 | DCP | TTCP | ---- | ---- | 1.50 | ---- | -- | 25 | 45 |
| 9 | DCP | TTCP | ---- | ---- | 1.67 | ---- | -- | 10 | 25 |
| 10 | DCPD | TTCP | ---- | ---- | 1.50 | PHA | 40 | 15 | 50 |
| 11 | DCPD | TTCP | ---- | ---- | 1.67 | ---- | -- | 5 | 35 |
| 12 | MCPM | ß-TCP | ---- | ---- | 1.00 | DCPD | 10 | 2 | 2 |
| 13 | MCPM | ß-TCP | ---- | ---- | 1.00 | ---- | -- | 1 | 2 |
| 14 | MCPM | TTCP | ---- | ---- | 1.00 | DCPD | 10 | 5 | 10 |
| 15 | DCPD | α-TCP | ---- | ---- | 1.33 | PHA | 40 | 15 | 60 |
| 16 | DCPD | TTCP | ---- | ---- | 1.33 | PHA | 40 | 15 | 60 |
| 17 | MCPM | α-TCP | ---- | ---- | 1.33 | PHA | 40 | 10 | 60 |
| 18 | MCPM | TTCP | ---- | ---- | 1.33 | PHA | 40 | 25 | 60 |
| 19 | MCPM | CaO | ---- | ---- | 1.33 | PHA | 40 | 10 | 30 |
| 20 | DCP | α-TCP | ---- | ---- | 1.33 | PHA | 40 | 10 | 55 |
| 21 | DCP | TTCP | ---- | ---- | 1.33 | PHA | 40 | 15 | 50 |
| 22 | DCP | ß-TCP | CaMgO$_2$ | ---- | 1.33 | ß-TCP | 40 | 15 | 25 |
| 23 | MCPM | α-TCP | CaMgO$_2$ | ---- | 1.28 | ß-TCP | 40 | 20 | 55 |
| 24 | DCP | α-TCP | CaMgO$_2$ | ---- | 1.28 | ß-TCP | 40 | 15 | 30 |
| 25 | DCP | TTCP | CaMgO$_2$ | ---- | 1.28 | ß-TCP | 40 | 20 | 30 |
| 26 | DCPD | TTCP | CaMgO$_2$ | ---- | 1.28 | ß-TCP | 40 | 20 | 45 |
| 27 | DCP | CaO | CaCO$_3$ | ---- | 2.00 | PHA | 40 | 6 | 50 |
| 28 | DCPD | CaO | CACO$_3$ | | 2.00 | PHA | 40 | 5 | 50 |
| 29 | DCP | Ca(OH)2 | CaCO$_3$ | ---- | 2.00 | PHA | 40 | 10 | 60 |
| 30 | NaWH | CIA | ---- | ---- | 1.50 | PHA | 40 | 20 | 55 |
| 31 | Rh | CIA | ---- | ---- | 1.50 | PHA | 40 | 10 | 60 |
| 32 | DCP | Rh | CaO | CaCO$_3$ 1.87 | | PHA | 40 | 10 | 45 |

EP 0 543 765 A1

| 1 | 2 | 3 | 4 | 5 | 6 | 7 | 8 | 9 | 10 |
|---|---|---|---|---|---|---|---|---|---|
| 33 | CaO | CaKPO$_4$ | Sp | ---- | 1.67 | PHA | 40 | 13 | 60 |
| 34 | DCPD | CaKPO4 | Sp | ---- | 1.33 | PHA | 40 | 15 | 35 |
| 35 | NaWH | CIA | DCP | ---- | 1.33 | PHA | 40 | 15 | 60 |
| 36 | MaWH | CIA | DCPD | ---- | 1.33 | PHA | 40 | 6 | 50 |
| 37 | NaWH | CIA | MCPM | ---- | 1.00 | DCPD | 40 | 4 | 13 |
| 38 | MCPM | CaO | ---- | ---- | 1.50 | SHA | 40 | 5 | 25 |
| 39 | DCP | TTCP | ---- | ---- | 1.50 | SHA | 40 | 10 | 40 |
| 40 | OCP | TTCP | ---- | ---- | 1.50 | PHA | 40 | 8 | 60 |
| 41 | MCPM | α-TCP | ---- | ---- | 1.33 | PHA | 40 | 20 | 50 |
| 42 | MCPM | α-TCP | CaMgO$_2$ | ---- | 1.28 | --- | --- | 13 | 25 |
| 43 | DCP | α-TCP | ---- | ---- | 1.33 | --- | --- | 20 | 45 |
| 44 | OCP | Ca(OH)$_2$ | ---- | ---- | 1.50 | PHA | 40 | 25 | 55 |
| 45 | MCPM | CaO | ---- | ---- | 1.50 | --- | --- | 8 | 30 |
| 46 | DCP | CaO | ---- | ---- | 1.50 | --- | --- | 15 | 20 |
| 47 | MCPM | CaO | CaMgO$_2$ | ---- | 1.28 | --- | --- | 10 | 50 |
| 48 | DCP | CaO | CaMgO$_2$ | ---- | 1.28 | --- | --- | 10 | 50 |
| 49 | DCPD | CaO | CaMgO$_2$ | ---- | 1.28 | --- | --- | 25 | 45 |
| 50 | MCPM | TTCP | ---- | ---- | 1.50 | --- | --- | 16 | 60 |
| 51 | MCPM | TTCP | ---- | ---- | 1.33 | --- | --- | 5 | 45 |
| 52 | MCPM | CaO | ---- | ---- | 1.33 | --- | --- | 8 | 35 |
| 53 | MCPM | CaO | ---- | ---- | 1.33 | SHA | 40 | 8 | 30 |
| 54 | DCP | TTCP | ---- | ---- | 1.33 | --- | --- | 15 | 25 |
| 55 | DCP | CaO | ---- | ---- | 1.33 | --- | --- | 7 | 13 |
| 56 | MCPM | ß-TCP | CaMgO$_2$ | ---- | 1.28 | --- | --- | 15 | 50 |
| 57 | DCP | ß-TCP | CaMgO$_2$ | ---- | 1.28 | --- | --- | 20 | 35 |
| 58 | NaWH | CIA | ---- | ---- | 1.50 | --- | --- | 20 | 50 |
| 59 | MCPM | CaO | CaCO$_3$ | ---- | 2.00 | --- | --- | 10 | 25 |
| 60 | CaHPO4 | Sp | CaO | ---- | 1.80 | --- | --- | 8 | 55 |

In a second embodiment, the cement bodies were prepared according to the powder blends shown in the aforegoing table, in a multiple teflon mould.

Each of the cement bodies had a cylindrical form with a diameter of 6 millimetres and a height of 12 millimetres.

Before setting, the moulds were warmed to about 35°C with a relative humidity of 100%, being maintained in these conditions for at least 20 hours.

The compression resistence was determined (being n comprised between 4 and 8) as well as the diametral tensile resistence (n comprised between 4 and 8).

Table 4 lists the blends with a compression resistence of 1,10 mPa or higher, satisfying these cements,

11

at least to a certain degree, the requisite relative to certain minimum compression and tensile resistence as desirable characteristics, even for applications without load.

It is therefore expected that both the formulations shown in the above table bearing the numbers 5, 22 to 26 and 42 as well as those shown with numerals 47 to 49, 56 and 57 concerning β-TCP and/or MgWH compositions will be more reabsorbible under conditions "in vivo" than the other formulations, for which reason, when having this group of cements available, the surgeon may choose calcium ortophosphate cements for use as more or less reabsorbible biomaterials according to the requisite corresponding to the choice of stability "in vivo or reabsorption in vivo" as desirable characteristics for the use of calcium ortophosphate cements as biomaterials, as previously stated.

By last, Table 5 is enclosed which corresponds to calcium ortophosphate cement formulations having a compression resistence of at least 1MPa after setting and after being stored for at least 20 hours with a relative humidity of 100%. In the table:

Column 1 refers to the formulation number.
Column 2 refers to the rate water/powder in weight.
Column 3 refers to compression resistence (MPa).
Column 4 refers to diametral tensile resistence (MPa).
Column 5 refers to porosity (%).

Table 5

| 1 | 2 | 3 | 4 | 5 | 1 | 2 | 3 | 4 | 5 |
|---|---|---|---|---|---|---|---|---|---|
| 1 | 0.6 | 2.9 | 0.5 | --- | 38 | 0.50 | 1.5 | 0.37 | --- |
| 2 | 0.6 | 2.3 | 0.4 | 62 | 41 | 0.45 | 6.3 | 1.5 | 41 |
| 4 | 0.5 | 2.7 | 0.5 | 62 | 42 | 0.55 | 12.0 | 2.8 | 47 |
| 5 | 0.3 | 1.0 | 0.3 | 43 | 43 | 0.30 | 9.9 | 2.1 | --- |
| 7 | 0.5 | 4.8 | 1.1 | 47 | 47 | 0.70 | 2.1 | 0.6 | 55 |
| 8 | 0.3 | 1.0 | 0.06 | 38 | 50 | 0.50 | 4.9 | 1.5 | 42 |
| 12 | 0.5 | 1.7 | 0.7 | 47 | 51 | 0.50 | 2.8 | 0.8 | 47 |
| 14 | 0.4 | 3.6 | 0.4 | 57 | 52 | 0.60 | 1.3 | 0.23 | 55 |
| 15 | 0.55 | 5.6 | 1.2 | 45 | 53 | 0.50 | 3.2 | 0.7 | 49 |
| 16 | 0.55 | 1.6 | 0.4 | 53 | 54 | 0.30 | 2.4 | 0.8 | 42 |
| 17 | 0.65 | 5.2 | 1.0 | 54 | 56 | 0.50 | 1.1 | 0.25 | 51 |
| 18 | 0.70 | 1.0 | 0.23 | 55 | 60 | 0.30 | 1.1 | 0.31 | 38 |
| 20 | 0.50 | 2.3 | 0.55 | 50 | | | | | |
| 21 | 0.55 | 1.2 | 0.27 | 52 | | | | | |
| 23 | 0.55 | 5.9 | 1.8 | 51 | | | | | |
| 30 | 0.35 | 1.2 | 0.27 | 42 | | | | | |
| 34 | 0.35 | 2.1 | ---- | --- | | | | | |
| 35 | 0.50 | 1.0 | 0.16 | 48 | | | | | |
| 36 | 0.50 | 1.3 | 0.28 | 50 | | | | | |

The process which is necessary to obtain the calcium phosphate cements of the invention is carried out as explained in this description, starting with the components which have been described in the aforegoing, which have to be ground and passed through a sieve up to the desired grain size which is approximately comprised between 5 and 10 micron.

Subsequently, they will be weighed according to the adequate quantity, after which the blend of powder products will be used to fill a bottle or wide mouth container, filling it up to a maximum of 25% of its capacity.

The bottle or container will be submitted to rotation on a roller for one hour or alternatively they will be shaken vigorously by hand for 5 minutes.

The mix will be placed afterwvards, in the desired portions, in bottles for its supply to surgeons or dentists together with capsules which contain the desired quantity of water or water solution.

Finally, the bottles will be sterilized as well as the capsules with its contents.

For its use in a hospital or health centre the powder product and the liquid will be supplied together with a glass plate or tile, to be mixed for one minute until a paste is obtained.

In case of injectable cements said paste will be placed in the added injection and the surgeon or dentist will proceed to the injection into the body.

In case of non injectable cements the paste will be applied on the body by means of a palette knife.

A more detailed explanation of the invention is not deemed necessary for the understanding of the same by experts in this field, in order to carry out the invention and to recognize its advantages.

The materials as well as its shape, size and disposition of the components or products will be variable Wherever this does not mean a change in the essentials on the invention.

The terms which have been used in this description will be taken in its amplest and non limiting meaning.

## REFERENCES

1.- F.C.M. Driessens and R.M.H. Verbeeck, Biominerals, CRC Press, Boca Raton, 1990.

2.- W.E. Brown and L.C. Chow, Dental restorative cement Pastes, US Patent 4.518.430 of May 21, 1985.

3.- Y.Fukase, E.D. Eanes, S. Takagi, L.C. Chow, and W.E. Brown Setting reactions and compressive strengths of calcium phosphate cements, J.Dent. Res. 69 1852-1856 (1990).

4.- H. Monma, A. Makishima, M. Mitomo and T. Ikegami, Hydraulic properties of the tricalcium phosphate-dicalcium phosphate mixture, Nippon-Seramikkusu-Kyokai-Gakujutsu-Ronbushi, 96 878-880 (1988).

5.- J. Lemaitre, A. Mirtschi and A. Mortoer, calcium phosphate cements for medical use: state of the art and perspectives of development, Sil. Ind. Ceram. Sci. Technol. 52 141-146 (1987).

6.- A. Mirtschi, J. Lemaitre and N. Terao, calcium phosphate cements: study of the β-tricalcium-phosphate-monocalcium phosphate system, Biomaterials 10 475-480 (1989).

7.- A.A. Mirtschi, J. Lemaitre and E. Munting, Calcium phosphate cements: action of setting regulators on the properties of the β-tricalcium phosphate-monocalcium phosphate cements, Biomaterials 10 634-638 (1989).

8.- H. Monma and T. Kanazawa, The hydration of α-Tricalcium phosphate, Yogio-Kyokai-Shi 84 209-213 (1976).

9. H. Monma, hydratation and hardeninf og brushite and monetite, Yogio-Kyokai-Shi 95 284-285 (1987).

10.- M.Ito, In vitro properties of a chitosan-bonded hydroxyapatite bone-filling paste, Biomaterials 12 41-45 (1991).

11.- H. Oonishi, Orthopaedic applications of hydroxyapatite, Biomaterials 12 171-178 (1991).

12.- H. Oonishi, H. Aoki, E. Tsuji and T. Mizukoshi, Biological and physical change of α-TCP bioactive bone cements three years after implantation, 2Nd Int.Symp. on Ceramics in Medicine, Heidelberg, 10-11, Sept. 1989.

13.- RW Phillips, Science of dental materials, Mosby, St. Louis, 1983.

14.- F.C.M. Driessens, Phystology of hard tissues in comparison with the solubility of synthetic calcium phosphates, Ann. NY Acad. Sci. 523 131-136 (1988).

15.- F.C.M. Driessens and R.M.H. Verbeeck, Relation between physico-chemical solubility and biodegradability of calcium phosphate, in: C de Putter, G.L. de Lange, K. de Groot and A.J.C. Lee (Eds.), Inplant materials in biofunction, Elseveir, Amsterdam, 1988, pp. 105-111.

16.- F.C.M. Driessens, Is the solubility product of synthetic calcium phosphates a good predictor of their biodegradability?, In: G. de With, R.A. Terpstra and R. Metselaar (Eds), Euroceramics, Elsevier, London, 1989, pp 3.48-3.52.

17.- M.M.A. Ramselaar, F.C.M. Driessens, W. Kalk, J.R. de Wijn and P.J. van Mullen, Biodegradation of four calcium phosphate ceramics: in vivo rates and tissue interactions, J. Mat. Sci. Mat. in Med. 2, 63-70 (1991).

18.- N.Nishimura, T. Yansamuro, T. Yakamura, Y. Taguchi, T. Kokubo and S. Yoshihara, A. novel bioactive bone cement based on CaO-SiO$_2$-P$_2$O$_5$-CaF$_2$glass, in: W. Bonfield, G. W. Hastings and K.E. Tanner (Eds.) Bioceramics, vol 4, Butterworht-Heinemann, London, 1991, p. 295-299.

**Claims**

1.- Process for the preparation of calcium phosphate cements and its application as bio-materials, characterized in that the calcium ortophosphate pastes to be used as biomaterials comprise a water mix of at least one reagent selected within the group comprising MCPM, DCP, DCPD, OCP, $\beta$-TCP, $\alpha$-TCP, TTCP, $Ca_4Mg_5(PO_4)_3$, $CaZn_2(PO_4)_2$, $CaKPO_4$, $CaNaPO_4$, $Ca_{10}Na(PO_4)_7$, $Ca_2PO_4Cl$ and $Ca_{10}(PO_4)_6Cl_2$ having said pastes the capacity to harden whenever before its setting they are submitted to 100% relative humidity.

2.- Process for the preparation of calcium phosphate cements and its application as bio-materials, according to claim 1, characterized for using pastes containing as additional reagent one or more compounds from the group $Ca(OH)_2$, CaO, $CaCO_3$, MgO, $MgCO_3$, $Mg(OH)_2$, ZnO, $ZnCO_3$ or Na, K, Mg or Zn ortophosphates.

3.- Process for the preparation of calcium phosphate cements and its application as bio-materials, according to the previous claims, characterized for using pastes which additionally contain other components comprising sodium, potassium, magnesium, zinc, calcium, biopolymers, organic or inorganic acids, synthetic polymers and growth factors used as accelerators, retarders o modifiers.

4.- Process for the preparation of calcium phosphate cements and its application as bio-materials, according to the previous claims, characterized for using pastes which further contain one or more crystal seeds choosen from the group DCPD, OCP, PHA, SHA, NCCA, NCDHA or bone minerals.

5.- Process for the preparation of calcium phosphate cements and its application as bio-materials, characterized for using pastes which have been prepared using water solutions or at least one soluble component of sodium, potassium, magnesium, zinc, calcium, biopolymers, organic acids, inorganic acids, synthetic polymers and growth factors.

6.- Process for the preparation of calcium phosphate cements and its application as bio-materials, characterized in that the components which to be used will be previously ground and submitted to sieving to obtain a grain size approximately comprised between 5 and 10 micron and afterwards they will be weighed to prepare the required quantities.

7.- Process for the preparation of calcium phosphate cements and its application as bio-materials, according to claim 6, characterized in that the blend of powder products will be put into a recipient to be filled up to 25,% of its capacity, submitting the same to rotation on a roller for 60 minutes or to vigorous shaking for 5 minutes, putting the blend afterwards in portions, adequate to its application, into a bottle for its supply to the specialists, together with capsules containing the desired quantity of water or water solution.

8.- Process for the preparation of calcium phosphate cements and its application as bio-materials, according the claims 5 and 6, characterized in that the bottles will be sterilized as well as the capsules and its contents, with the possibility of supplying to health centers the powder product and the liquid together on a glass plate or on tiles, to be mixed for a few minutes to obtain a paste.

9.- Process for the preparation of calcium phosphate cements and its application as bio-materials, according to claim 8, characterized in that in case of injectable cements, the paste which has been obtained will be put into the added injection for its injection to the body and in case of non injectable cements, the paste will be applied by means of a palete knife.

European Patent
Office

**EUROPEAN SEARCH REPORT**

Application Number

EP 92 50 0139

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | US-A-4 518 430 (W. BROWN)<br>* column 6, line 11 - line 14 *<br>* column 8, line 60 - column 9, line 42; claims * | 1 | A61L25/00<br>A61K6/033 |
| X | WO-A-9 000 892 (NITTA GELATIN)<br>* page 8, paragraph 1 *<br>* page 9, paragraph 3 *<br>* page 14, last paragraph - page 15; claims * | 3,5,6 | |
| X | EP-A-0 298 501 (ASAHI KOGAKU KOGYO KK)<br>* page 4, line 27 - line 40; claims; tables * | 3,5 | |
| X | DATABASE WPIL<br>Week 8946,<br>Derwent Publications Ltd., London, GB;<br>AN 89-336055<br>& JP-A-1 250 264 (LION CORP.)<br>* abstract * | 1-3 | |
| A | US-A-3 873 327 (E. DUFF)<br>* claims; example * | 2 | TECHNICAL FIELDS SEARCHED (Int. Cl.5)<br><br>A61L<br>A61K |
| A | DE-A-4 016 135 (TOKUYAMA SODA K.K.)<br>* page 2, line 38 - line 42 *<br>* page 3, line 20 - line 24; claims * | 8 | |
| A | US-A-4 911 641 (S. DETSCH)<br>* column 4, line 64 - column 5, line 15; figure 8 * | 9 | |

The present search report has been drawn up for all claims

| Place of search | Date of completion of the search | Examiner |
|---|---|---|
| THE HAGUE | 03 MARCH 1993 | G.COUSINS- VAN STEEN |

EPO FORM 1503 03.82 (P0401)